(19) Europäisches Patentamt
European Patent Office
Office européen des brevets

(11) Publication number: **0 457 559 A2**

# (12) EUROPEAN PATENT APPLICATION

(21) Application number: **91304345.1**

(51) Int. Cl.[5]: **C07C 215/30**, C07D 333/20

(22) Date of filing: **15.05.91**

(30) Priority: **17.05.90 US 524512**

(43) Date of publication of application: **21.11.91 Bulletin 91/47**

(84) Designated Contracting States: **AT BE CH DE DK ES FR GB GR IT LI NL SE**

(71) Applicant: **ELI LILLY AND COMPANY**
**Lilly Corporate Center**
**Indianapolis Indiana 46285 (US)**

(72) Inventor: **Staszak, Michael Alexander**
**4515 North Lakeridge Drive**
**Indianapolis, Indiana 46234 (US)**
Inventor: **Staten, Gilbert Stanley**
**74 Staten Lane**
**Camby, Indiana 46113 (US)**
Inventor: **Weigel, Leland Otto**
**7501 Chatterton Drive**
**Indianapolis, Indiana 46254 (US)**

(74) Representative: **Tapping, Kenneth George et al**
**Lilly Industries Limited Patent Department Erl Wood Manor**
**Windlesham Surrey, GU20 6PH (GB)**

(54) **Chiral synthesis of 1-aryl-3-aminopropan-1-ols.**

(57) This invention is directed to a process for preparing chiral 1-aryl-3-amino-propan-1-ols. These compounds are important intermediates in the synthesis of pharmacologically active compounds.



Jouve, 18, rue Saint-Denis, 75001 PARIS

Background of the Invention

Recently, the relationship between monoamine uptake and a variety of diseases and conditions has been appreciated and investigated. For example, the hydrochloride salt of fluoxetine (dl-N-methyl-γ-[4-(trifluoromethyl)phenoxy]benzenepropanamine) is a selective serotonin (5-hydroxytryptamine) uptake inhibitor presently available for the treatment of depression. Similarly, tomoxetine hydrochloride ((-)-N-methyl-y-(2-methylphenoxy)benzenepropanamine hydrochloride) is a selective inhibitor of norepinephrine uptake being investigated clinically for its antidepressant activity. These compounds are among those taught in U.S. Patents Number 4,313,896 and 4,314,081 and EPA 273658 (1988) as being potent but selective blockers of the uptake of a particular monoamine.

The compounds taught in those references are disclosed as useful for treating a variety of disorders which have been linked to decreased neurotransmission of serotonin and/or norepinephrine in mammals including obesity, depression, alcoholism, pain, loss of memory, anxiety, smoking, and the like.

Although the compounds disclosed in said references can be prepared in several ways as described therein, a particularly useful synthetic reaction common to the compounds of all three references is a Mannich reaction on an appropriate ketone to afford a 1-aryl-3-aminopropan-1-ol (Mannich Base). The Mannich Base afforded by this reaction is further reacted with appropriate compounds under described conditions to provide the desired compounds taught in those references.

Each of these references teaches that the compounds disclosed therein possess at least one asymmetric carbon atom. As such, the compounds can exist as the individual stereoisomers as well as the racemic mixtures. The references state that the individual optically active isomers may be obtained by resolving the racemate. Such resolution being accomplished by methods well known in the art, particularly by forming salts with optically active acids and separating the salts by crystallization.

EPA 273658 further discloses that optically active isomers, Examples 14, 38 and 39, may be prepared from their respective optically active precursors by the procedures described therein. The specific procedures used to arrive at the individual isomers of Examples 14, 38 and 39 are not disclosed.

Chromatography, as a generally known means of resolving optically active isomers, is also disclosed in EPA 273658. A discussion on chromatographic separation of chiral fluoxetine, tomoxetine, and a particular antidepressant taught in EPA 273658 using chiral chromatographic techniques is set forth in Bopp, et al., "Practical Considerations for Chiral separations of Pharmaceutical Compounds," Liquid and Gas Chromatography, 6 (6), (1988).

The chemical literature in recent years has reflected an increasing interest in chiral starting materials and reagents for the synthesis of optically active compounds. There are two general approaches to the enantioselective reduction of carbonyl groups: (i) the use of aluminum or boron hydrides having chiral ligands, and (ii) transfer of chirality by exchange of a hydride ion attached to a chiral center. The "transfer of chirality" approach requires activation of the carbon-hydrogen bond, typically by a metal atom in a β-position or by an alkoxide group in an α-position. The use of chiral lithium aluminum hydrides has received the most attention and investigation.

Cervinka and associates have studied asymmetric reduction of achiral ketones using lithium aluminum hydride (LAH) complexed with alkaloids and related amino alcohols, Haubenstock, Top. stereochem., 14, 231 at 262 (1982). Landor and coworkers studied chiral reductions using LAH complexed with monosaccharides and derivatives thereof, Yamaguchi, Mosher and Pohland, J. Amer. Chem. soc., 94, 9254 (1972).

Asymmetric reductions of achiral ketones with a chiral reducing agent prepared by reacting 2 to 3 equivalents of (2s, 3R,)-(+)-4-dimethylamino-1,2-diphenyl-3-methyl-2-butanol with LAH was studied by Yamaguchi and Mosher, J. Org. Chem., 38 (10), 1870 (1973); and Yamaguchi, Mosher and Pohland, J. Amer. Chem. soc. 94, 9254 (1972). Achiral ketone substrates used in these studies included benzaldehyde, acetophenone, phenyl trifluoromethyl ketone, propiophenone, n-butyrophenone, phenyl isopropyl ketone, phenyl tert-butyl ketone and methyl tert-butyl ketone.

In Brinkmeyer and Kapoor, J. Amer. Chem. soc., 99, 8339 (1977) and Johnson, et al., J. Amer. Chem. soc., 99, 8341 (1977), the asymmetric reduction of aliphatic acetylenic ketones with LAH complexed with (2s,3R)-(+)-4-dimethylamino-3-methyl-1,2-diphenyl-2-butanol is described. Cohen et. al., J. Org. Chem., 45 (4), 582 (1980) describes the asymmetric reductions of α,β-acetylenic ketones and acetophenone using a freshly prepared complex afforded by reacting LAH and certain optically active 1,3-amino alcohols. Andrisano, et al., in Tetrahedron, 29, 193-196 (1973) describes the asymmetric reduction of achiral β-dialkyl-aminopropiophenones with LAH partly complexed with (-)-menthol.

In Cohen, et al., and Kabuto, et al., J. Org. Chem., 42 (10), 1742 (1977) the use of (2R,3S)-(-)-4-dimethylamino-3-methyl-1,2-diphenyl-2-butanol in assymetric reduction of acetylenic ketones and cis-2-acetoxy-6-phenylcyclohexanone and 1-acetoxy-2-hydroxy-3-phenylcyclohexanone is described.

Although acetylenic ketones are an exception, generally, the production of optically active synthetic intermediates from achiral staring materials by means of asymmetric reduction has been of more theoretical than practical interest. In general, laboratory scale asymmetric reductions of aryl ketones and acetylenic ketones using LAH complexed with a dextro carbinol base (2S,3R)-(+)-4-dimethylamino-1,2-diphenyl-3-methyl-2-butanol (referred at times as "dcb") or a levo carbinol base (2R,3S)-(-)-4-dimethyl-amino-1,2-diphenyl-3-methyl-2-butanol (referred to at times herein as "lcb") affords chiral alcohols having an enantiomeric excess (ee) up to about 60%. Only the acetylenic ketones reportedly afford ee's of above 70%. Even in those cases where the ee's are reportedly above 70%, there is little or no predictably associated with obtaining such ee values.

Asymmetric synthesis reactions utilizing LAH complexed with dcb or lcb are notoriously capricious. Numerous factors including temperature, ratio of LAH: chiral ligand: substrate, solvent, age of LAH complex, structural features present in the substrate, structural features present in the chiral ligand and the like impact on the reaction and yields afforded from such a reaction.

Even the preparation of the reduction complex has reportedly had a significant effect on the optical yield and the configuration of the enantiomer obtained in excess, Cegla, Polish Journal of Chemistry, 59, 1265 (1985).

Unexpectedly, it has been discovered that chiral 1-aryl-3-aminopropan-1-ols having an enantiomeric excess of greater than 70% can be afforded by reducing the corresponding Mannich ketone with a complex of LAH and lcb.

It is one object of the present invention to provide a commercially practical process for the asymmetric reduction of achiral aromatic β-amino ketones to chiral aromatic β-amino alcohols in high enantiomeric excess of the desired enantiomer.

## Summary of the Invention

In fulfillment of the above and other objects, this invention provides a process for preparing a chiral β-amino alcohol having the formula

$$\begin{array}{c}\phantom{Ar-}OH\phantom{-CH_2-CH_2-NR^1R^2}\\ \phantom{Ar-}|\phantom{-CH_2-CH_2-NR^1R^2}\\ Ar-CH-CH_2-CH_2-NR^1R^2\end{array}$$

wherein:

Ar is phenyl or thienyl; and $R^1$ and $R^2$ are independently selected from $C_1$-$C_3$ alkyl and phen($C_1$-$C_3$)-alkyl comprising reacting an achiral Mannich ketone having the formula

$$\begin{array}{c}\phantom{Ar-}O\phantom{-CH_2-CH_2-NR^1R^2}\\ \phantom{Ar-}\|\phantom{-CH_2-CH_2-NR^1R^2}\\ Ar-C-CH_2-CH_2-NR^1R^2\end{array}$$

wherein:

Ar, $R^1$ and $R^2$ are as previously defined with a lithium aluminum hydride and (2R,3S)-(-)-4-dimethylamino-1,2-diphenyl-3-methyl-2-butanol complex in an inert organic solvent and recovering therefrom said chiral β-amino alcohol.

The present invention also provides a process for preparing (S)-(+)-N-methyl-3-(1-naphthalenyloxy)-3-(2-thienyl)propanamine maleate comprising:

a) reacting 2-acetylthiophene with dimethylamine hydrochloride and formaldehyde to afford 3-dimethylamino-1-(2-thienyl)-1-propanone which is recovered as an acid addition salt; and

b) reducing the compound afforded in (a) with a suspension complex of LAH/lcb to afford (s)-(-)-N,N-dimethyl-3-(2-thienyl)-3-hydroxy-propanamine which is recovered as an acid addition salt; and

c) reacting the compound afforded in (b) with a 1-halonaphthalene to afford (S)-(+)-N,N-dimethyl-3-(1-naphthalenyloxy)-3-(2-thienyl)propanamine which is recovered as an acid addition salt; and

d) reacting the compound afforded in (c) with ROCOC1 where R is phenyl, benzyl, ethyl, vinyl, 1-chloroethyl or 2,2,2-trichloroethyl, in the presence of a releasing reagent to afford (S)-(-)-N-methyl-3-(1-naphthalenyloxy)-3-(2-thienyl)-propanamine which is recovered as an acid addition salt; and

e) reacting the compound afforded in (d) with maleic acid in ethyl acetate to afford (S)-(+)-N-methyl-3-(1-naphthalenyloxy)-3-(2-thienyl)-propanamine which is recovered as the maleate salt.

Detailed Description of the Invention

The term "$C_1$-$C_3$alkyl", as used herein, means a straight or branched alkyl chain having from 1 to 3 carbon atoms. Such alkyl groups are methyl, ethyl, n-propyl, and isopropyl. The term "halo" means chloro, fluoro, bromo, or iodo. The term "releasing reagent" means zinc, formic acid, acetic acid, $NaH_2PO_4$, NaOH, KOH, LiOH or mixtures thereof.

Although the entire scope of variables taught herein are believed operable, the present invention does have preferred aspects. In the above Formula I and II, Ar preferably is thienyl and $R_1$ and $R_2$ are preferably $C_1$-$C_3$alkyl, and especially methyl.

The chiral ligand or complexing agent lcb is prepared from propiophenone, formaldehyde and dimethylamine using standard Mannich reaction conditions to afford the corresponding Mannich Base. The Mannich Base is then reacted with a benzylmagnesium chloride Grignard reagent in an ether solvent to afford 4-dimethylamino-1,2-diphenyl-3-methyl-2-butanol racemic mixture. This d,l mixture is then resolved with (-)-camphorosulfonic acid in ethanol to afford dcb. The desired lcb isomer is efficiently recovered from the mother liquors of said dcb resolution by resolution with the same acid, that is, (-)-camphorosulfonic acid in acetone. Further details regarding the preparation and isolation are found in Pohland, sullivan, J. Amer. Chem. soc., 77, 3400 (1955) incorporated herein by reference.

Similar to the Mannich reaction described above for obtaining lcb, the Mannich ketones used as reactants in the enantioselective synthesis of the present invention are prepared by reacting acetophenone or 2-acetylthiophene with formaldehyde and dimethylamine under standard Mannich reaction conditions.

The LAH:lcb complex is generally prepared by the addition of dry lcb to $LAH(THF)_2$ in a ratio of from about 1.8:1 to 2.7:1, preferably from about 2.0 to 2.5:1 in an aromatic solvent preferably toluene, at from about 0°C to about -40°C, preferably from about -25°C to about -30°C. The addition should be completed in less than ten minutes and carried out under an inert atmosphere.

Optionally, a small amount of powdered molecular sieves may be added to the $LAH(THF)_2$ in an aromatic solvent prior to the addition of dry lcb. It is believed the sieves assist in affording the "insoluble" form of the reduction complex, but are not required. Preferably, molecular sieves are simply ground to a powder with a mortar and pestle. About one spatula of the powdered sieves is added, when used. Further details regarding the insoluable and soluable forms of the reduction complex are in J. Org. Chem., 38, 1870-1877 (1973).

The complex should not be allowed to age longer than ten minutes as a reversal of stereochemistry will result when utilized in the reduction reaction. Further, lower optical yields generally, and particularly lower ee's, are afforded. The so-called "soluble" complex or homogeneous solution of the reduction complex is also not desirable for use in the present invention as the optical yields afforded are at best moderate and the ratio of optical isomers is not as advantageous.

Immediately after the addition is completed, the insoluble complex is cooled to a temperature from about -60°C to about -75°C and a dry solution of the Mannich ketone in an aromatic solvent, preferably toluene, is added in less than 10 minutes. The temperature is maintained between about -65°C and about -70°C during the course of the reaction which is carried out under an inert atmosphere for from about 13 to 20 hours. The reaction mixture is then quenched by the addition of sodium sulfate, filtered, and washed with hot THF, and then concentrated in vacuo. The desired β-amino alcohol is then isolated by addition of hexane and extraction into a 1:1 $C_1$-$C_3$ alkanol: water mixture, preferably aqueous methanol, concentrated and filtered.

Difficulties typically associated with use of LAH complexes are avoided in the process of the present invention. Most notable among these are highly efficient upgrades in the ee of the chiral β-amino alcohol are accomplished in a very simple manner. For example, isolated chiral β-amino alcohol is stirred in an aqueous $C_1$-$C_3$ alkanol mixture (1:1 v/v) for 30-60 minutes at room temperature, and then filtered and dried. Further, simple extraction efficiently separates the lcb chiral ligand from the desired chiral β-amino alcohol. Purified lcb ligand is recovered by simply cooling the extraction solution to between about -35°C to about 0°C and filtering the solution. Concentration of the filtrate, recooling, and filtration provides a second crop with a total recovery of chiral lcb ligand of greater than 80%. It will also be appreciated that by using aromatics, and particularly toluene, as a reaction solvent, rather than diethyl ether as is typically shown in the references, a greater margin of safety is provided as $LAH(THF)_2$ in toluene does not easily ignite. The process of the present invention permits large scale reductions (greater than 1 kg to be prepared) either by means of a batch reaction or in a continuous operation. In large scale operation, that is 22 L capacity batch equipment, the ee's fall to about 75%. However, higher ee's (greater than 80%) are obtained when the LAH:lcb complex formation reaction is carried out in a low pressure stirred reactor.

As stated above, the process of the present invention is used to prepare s-(+)-N-methyl-3-(1-naphthalenyloxy)-3-(2-thienyl) propanamine maleate. The intermediates used in this process are recovered as acid addition salts. Suitable acid addition salts are hydrochloride, hydrobromide, tartrate, mesylate, oxalate, and the like. Preferably, both 3-dimethylamino-1-(2-thienyl)-1-propanone and (S)-(-)-N,N-dimethyl-3-(2-thienyl)-3-hydroxypropanamine are recovered as the hydrochloride salt and (S)-(+)-N,N-dimethyl-3-(1-naphthalenyloxy)-3-(2-thienyl)propanamine and (S)-(-)-N-methyl-3-(1-naphthalenyloxy)-3-(2-thienyl)-propanamine are recovered as the oxalate salt.

The (s)-hydroxy intermediate prepared by the above enantioselective reduction is treated with an alkali metal hydride, preferably sodium hydride or potassium hydride, to form the corresponding alkali metal salt, which is then reacted with a naphthalene compound containing a good leaving group to provide the corresponding 3-naphthyloxy-3-(2-thienyl)propanamine. Good leaving groups are the halonaphthalenes, preferably fluoronaphthalene.

This reaction is carried out by combining approximately equimolar quantities with the (S)-hydroxy compound to provide the corresponding alkali metal salt. The compound is then reacted with an equimolar quantity to slight excess of the halonaphthalene compound. The reaction is conducted in a suitable aprotic solvent such as dimethylsulfoxide, N,N-dimethylacetamide and related solvents. The reaction is substantially complete after about 10 minutes to about 24 hours when conducted at a temperature in the range of about 25°C to about 150°C. More preferably, the reaction mixture will be completed within about 30 minutes to about 6 hours when conducted at a temperature in the range of about 75°C to about 125°C. The naphthyloxy intermediate may be isolated by standard conditions. Typically, the mixture is diluted with water and extracted with a water immiscible organic solvent such as diethyl ether, ethyl acetate, chloroform and the like. The organic extracts are typically combined and dried. Following evaporation of the organic solvent the isolated residue may be further purified, if desired, by standard techniques such as crystallization from common solvents, or chromatography over solid supports such as silica gel or alumina.

For the demethylation step, a compound ROCOC1, where R is phenyl, benzyl, ethyl, vinyl, 1-chloroethyl or 2,2,2-trichloroethyl is reacted with the N,N-dimethylpropanamine in the presence of a releasing reagent to provide the corresponding intermediate which is hydrolyzed in base to provide the desired N-methylpropanamine. Releasing reagents useful in this reaction include zinc, formic acid, acetic acid, $NaH_2PO_4$, NaOH, KOH, LiOH or mixtures thereof. Preferably, R is 2,2,2-trichloro-ethyl and the releasing reagent is a mixture of zinc and formic acid.

The maleate salt is prepared by dissolving the oxalate salt in purified water and extraction with ethyl acetate to regenerate the free base. The free base ethyl acetate solution is then treated with maleic acid and the maleate salt recovered.

The acid addition salts of the intermediates are typically formed by reacting propanamine with an equimolar or excess amount of acid. The reactants are generally combined in a mutual solvent such as diethyl ether or benzene, and the salt normally precipitates out of solution within about one hour to 10 days, and can be isolated by filtration.

As stated above the compounds prepared by the process of the present invention are useful intermediates in the preparation of pharmacologically active compounds. Said compounds are selective serotonin and/or norepinephrine uptake inhibitors which are useful for the treatment of depression, anxiety, appetite suppression, obesity, pain, loss of memory, smoking, and the like. Further details regarding the compounds utility and methods of preparing these compounds are discussed in detail in U.S. Patent Numbers 4,313,896 and 4,314,081 and EPA 273658 all three of which are incorporated by reference herein in their entirety.

The following Examples further illustrate the process of the present invention. These Examples are not intended to be limiting to the scope of the invention in any respect and should not be so construed. Chiral assays were performed on free amines by 300 MHz $^1H$ NMR analysis in the presence of R-(-)2,2,2-trifluoro-1-(9-anthryl)ethanol (TAE) as shift reagent in $CDC1_3$. Direct chiral HPLC assays were performed on a Chiralcel® OD column using 0.2% diethylamine in 10% isopropanol-hexane at 230 or 254 nm at 1.00 ml/min.

## Example 1

N,N-Dimethyl-3-(1-napthalenyloxy)-3-(2-thienyl)propanamine oxalate

A. 3 -Dimethylamino-1-(2-thienyl)-1-propanone hydrochloride

A mixture of 2-acetylthiophene 2.274 kg, 18.0 mol), dimethylamine hydrochloride (1.91 kg, 23.4 mol), paraformaldehyde (0.8108 kg, 9.0 mol), and 37.5 ml of concentrated hydrochloric acid in ethanol (5.6 L) is refluxed for twenty hours. The mixture is cooled to 35°C slowly then chilled to 0-5°C. The solid is collected by vacuum

filtration , washed twice with 1 L of ethanol, and vacuum dried at room temperature for 49 hours to afford 3.391 kg (85.7%) of 3-dimethylamino-1-(2-thienyl)-1-propanone hydrochloride. mp = 174-176°C

Analysis

| | |
|---|---|
| Theory: | C, 49.20; H, 6.42; N, 6.37; |
| Found: | C, 49.43; H, 6.35; N, 6.12. |
| Mass Spec: | $M^+$ (183, 182, 168, 111) |

B. 1-β-hydroxy-1-(2-thienyl)-3-dimethylaminopropane

To 3.2 L of toluene is added 953.6 g of (2R,3S)-(-)-4-dimethylamino-1,2-diphenyl-3-methyl-2-butanol and azeotropically dried to 112°C, then cooled to 30°C and used immediately as described below.

A 5 L flask is charged with 1.6 L of toluene and 344.8 g of the hydrochloride salt prepared above in step A. To the solution is added, dropwise, 1.6 L of 1N NaOH over 5-6 minutes at room temperature. The mixture is stirred for 30 minutes after the addition is complete and allowed to settle for 10 minutes. The resulting layers are separated and the organic layer (free base of the hydrochloride salt) washed with 100 ml of purified water. The organic layer is separated and azeotropically dried to 112°C in toluene, then rapidly chilled to -70°C for immediate use as described below.

A dry 22 L, 5-neck flask equipped with a mechanical stirrer, low temperature thermometer, condenser, nitrogen purge, cooling bath and two 2 L addition funnels is purged with nitrogen. The flask is charged with 3.2 L of toluene. To the flask is added 1.6 L of 1N $LiA1H_4(THF)_2$ in toluene. The mixture is chilled to -25°C and dry (2R,3S)-(-)-4-dimethylamino-1,2-diphenyl-3-methyl-2-butanol, prepared above, is added over 15 minutes. The reaction mixture is chilled to about -63°C and the cold free base solution, prepared above, is streamed in over 8-9 minutes. The reaction mixture is stirred at -75°C for 20 hours.

The reaction mixture is then warmed to 0°C and 61 ml of purified $H_2O$ is dripped in over 20 minutes, then stirred for 10 minutes. Over 10 minutes, 61 ml of 15% NaOH prepared from solid NaOH and purified water is dripped in and the mixture stirred for 10 minutes. Purified $H_2O$, 183 ml, is dripped in and the mixture is then filtered through diatomaceous earth (HYFLO®). The organic material is washed twice with 500 ml of THF and the filtrate evaporated at room temperature to afford 1.32 kg of a white solid. To the solid is added 1.4 L of hexanes and allowed to sit for 20 hours. The solution is extracted eight times with 50% $CH_3OH$ prepare from purifed water and $CH_3OH$. To the combined extracts is added 500 ml of hexanes and the mixture stirred, and then separated. The residual aqueous $CH_3OH$ is evaporated off at room temperature to afford 254 g of crude product.

NMR evidenced 85.8% (-) isomer and 14.2% (+) isomer as judged from N-methyl singlet.

The crude product (254 g) is dissolved in 500 ml of $CH_3OH$ at 35°C and 1 L of purified water is slowly added. The solution is seeded with (S)-(-)-1-β-hydroxy-1-(2-thienyl)-3-dimethylaminopropane and allowed to sit undisturbed while slowly cooling to 20-25°C. The mixture is placed in the freezer at -15°C for $4\frac{1}{2}$ hours, removed and vacuum filtered. The filtrate is washed twice with 100 ml at 50% $CH_3OH$ and vaccum dried at 20-25°C to afford 171.6 g of the sub-titled compound. mp = 78-80°C

NMR evidenced only the (-) isomer as judged from the N-methyl singlet.

Optical Rotation: $[\alpha]_D^{25}$ (C=1, MeOH) = -7.6°

HPLC showed 98.7% purity of the (-) isomer.

C. N-N-dimethyl-3-(1-naphthalenyloxy)-3-(2-thienyl)propanaminoxalate

To a 5 liter round-bottomed flask filtered with a thermometer, cooling bath, thermowatch and mechanical stirrer and under a nitrogen atmosphere is added 1.4 liter of dimethylsulfoxide (DMSO). To the DMSO is added 1-β-hydroxy-1-(2-thienyl)-3-dimethylaminopropane (400 g, 2.16 mol). (Several lots of the dimethylaminopropane prepared substantially as described above in step A and B are combined to afford the 400 g amount). The mixture is stirred for about 5 minutes. Over 90 minutes four portions (20.5 g each) of 60% NaH is added while maintaining the temperature between 20-30°C. 1-Fluoronaphthalene (284.4 ml, 2.59 mol) is added and the reaction mixture stirred for 4.5 hours. The reaction mixture was slowly heated to 43-44°C and heated for 64 hours. Temperature fluctuations are prevented by the thermowatch.

The reaction mixture is cooled to 25-27°C and a further 6.0 g of NaH added to regain the 0.95 eq. level for NaH. The reaction mixture is stirred for 5 hours and then heated to 43-44°C for 24 hours.

The reaction mixture is cooled to 25°C and poured into 5.5 liter of purified water. While controlling the temperature at 25-30°C by the rate of addition, 360 ml of concentrated HC1 is added. Hexanes (2 liters) are added and the reaction mixture stirred for 10 minutes and then allowed to sit for 10 minutes. The resulting layers are separated and 308 ml of 50% NaOH is dripped into the aqueous layer. The aqueous layer is then extracted four times with 2 liters of $CH_2C1_2$. The combined extracts are washed with 500 ml of water, and then separated. The organic layer is stirred with $Na_2SO_4$, then filtered and evaporated at 40°C to afford 681 g of an oil.

The oil is dissolved into 2.4 liters of methanol and oxalic acid (194.4 g, 1 eq) is added and then 6.4 liters of ethyl acetate. The reaction mixture is warmed to reflux and the methanol slowly distilled off while adding back one equivalent amount (by volume) of ethyl acetate. After precipitation of an organic material, the reaction mixture is removed from the heat and 5.2 liters of ethyl acetate is added while allowing the mixture to cool slowly without stirring.

The solids are chilled in a freezer to -15°C for 2 hours, filtered then vacuum dried at 27-30°C to afford 726 g of the sub-titled compound. mp = 151-152°C Optical Rotation: $[\alpha]_D^{25}$ (C=1, MeOH) = + 105.34°

| Analysis | C | H | N |
|---|---|---|---|
| Calculated: | 62.67 | 6.01 | 3.40 |
| Found: | 62.42 | 5.85 | 3.24 |

D. N-methyl-3-(1-naphthalenyloxy)-3-(2-thienyl)propanamine oxalate.

A solution of 726 g of N,N-dimethyl-3-(1-naphthalenyloxy)-3-(2-thienyl)-propanamine oxalate, 5.5 liters of purified water and 2.48 liters of 2 N sodium hydroxide under nitrogen is extracted 3 times with 4 liter portions of toluene. The toluene layers are combined and dried with $Na_2SO_4$, then filtered and washed with 2 liters of toluene.

To the solution is added 42.8 g of Proton Sponge® and 1.44 liters of toluene is distilled off. The reaction mixture is cooled to 25°C and 408 ml of 2,2,2-trichloroethyl chloroformate is added. The reaction mixture is heated slowly (1 hour) to 80°C and stirred at 75-80°C for 1 hour. An additional 88 ml of 2,2,2-trichloroethyl chloroformate is added and stirring continued while slowly cooling overnight.

The reaction mixture is washed with 3.4 liters of 1 N HC1 and then back extracted with 3.4 liters of toluene. The toluene layers are combined, dried with $Na_2SO_4$, filtered, rinsed with 2 liters of toluene and evaporated at 40°C to an oil (1.32 kg). The oil is dissolved in 6 liters of dimethylformamide (DMF) and chilled to 0°C in an ice/acetone bath.

Active zinc dust is prepared by washing 600 g of zinc dust twice with 1.5 liter portions of 10% HC1 which is prepared from mixing 2.34 liters of purified water with 678 ml of 12 N HC1. The zinc is then washed 3 times with 1.5 liter portions of methanol, and then 1.5 liters of ethanol, vacuum filtered under nitrogen, washed twice with 250 ml portions of ethanol and then vacuum dried under nitrogen.

To the chilled DMF solution is added 152 ml of formic acid and then 325.5 g of the active zinc. The reaction mixture is stirred for 4.5 hours at 0-5°C. The zinc is filtered off and rinsed twice with 250 ml portions of DMF. The DMF solutions are combined and diluted with 8 liters of purified water then the pH adjusted to 11 with $NH_4OH$. The solution is then extracted 6 times with 2 liter portions of ethyl acetate. The extracts are combined and dried over $Na_2SO_4$ and stored overnight at -5°C.

The extracts are filtered, washed twice with 1 liter portions of ethyl acetate and evaporated to an oil. The oil is then dissolved in 12 liters of ethyl acetate and 179.2 g of oxalic acid is added with stirring. The mixture is placed in an ice/acetone bath for 2 hours with occasional stirring. The mixture is then vacuum filtered, washed twice with 500 ml portions of ethyl acetate and vacuum filtered overnight. The solids are vacuum dried at 20-25°C for 72 hours to afford 628 g of the sub-titled compound. mp = 149-150°C

Chiral HPLC showed 97.5% of the S-(-) isomer and 2.5% of the antipode isomer. ee = 95%

Optical Rotation: $[\alpha]_D^{25}$ (C=1, MeOH) = + 84°

<u>Example 2</u>

N-Methyl-N-benzyl-3-(β-hydroxy)-3-(2-thienyl)propanamine

By substantially following the procedures described above in Example 1 Step A and B except using N-methylbenzylamine in step A and 1.1 equivalents of LAH and 2.3 equivalents of Icb in Step B the title compound

is afforded. NMR evidenced an ee of 84% as judged from the N-methyl singlet.

Example 3

N,N-Dimethyl-3-(β-hydroxy)-3-phenylpropanamine

By substantially following the procedures described above in Example 1, Steps A and B except using acetophenone, in step A and 1.1 equivalents of LAH and 2.5 equivalents of Icb in Step B the title compound is afforded. NMR evidenced ee of 86% as judged from the N-methyl singlet.

Example 4

Preparation of 1-β-hydroxy-1-(2-thienyl)-3-dimethylaminopropane

3-Dimethylamino-1-(2-thienyl)-1-propanone hydrochloride is prepared substantially as described in Example 1, step A.

To 5.0 g (0.0227 mol) of the hydrochloride salt under nitrogen is added 58 ml of toluene and 4.9 ml of 5 N NaOH is dripped in at 20-25°C. The reaction mixture is stirred for 30 minutes and then allowed to sit for 45 minutes. The resulting layers are separated and the toluene layer dried over 40 g of 4A sieves. The mixture is stirred for 2 hours and then allowed to sit for 18 hours over the sieves to afford the free base 3-dimethylamino-1-(2-thienyl)-1-propanone. The free base is cooled to -70°C under nitrogen for use below.

Dry (2R,3S)-(-)-4-dymethylamino-1,2-diphenyl-3-methyl-2-butanol (15.63 g, 0.0552 mol) is dissolved in 160 ml of toluene and stored under a nitrogen blanket at -70°C.

To 25.1 ml of 1M $LiA1H_4(THF)_2$ is added 46.0 ml of toluene and 1 spatula of powdered 4A sieves and the mixture cooled to -32°C. The cooled solution of (2R,3S)-(-)-4-methylamino-1,2-diphenyl-3-methyl-2-butanol is added dropwise over 7 minutes. The thick slurry is allowed to stir at -32 to -37°C for 4 minutes and then cooled to -62°C. The cold free base solution is added over 7 minutes. The resulting mixture is allowed to stir overnight under nitrogen at -76°C.

The mixture is allowed to warm to 0°C and then sequentially quenched with 0.95 ml of purified water, 0.95 ml of 15% NaOH and 2.85 ml of purified water. The resulting solution is allowed to stir for one hour and then filtered through diatomaceous earth (HYFLO®). The extracts are washed twice with 25 ml portions of THF. The filtrate is concentrated under vacuum to an oil. The oil is added to 25 ml of hexanes and the solution extracted eight times with 25 ml portions of 50% aqueous methanol. The combined extracts are back washed with 25 ml of hexanes and then concentrated under vacuum. The solids are allowed to pull under vacuum over the weekend to afford 3.59 g of a white crystalline solid. NMR evidenced an ee of 87.8% as judged from the N-methyl singlet.

## Claims

1. A process for preparing a chiral ß-amino alcohol having the formula

$$\begin{array}{l} \quad\ \ OH \\ \quad\ \ \vert \\ Ar{-}CH{-}CH_2{-}CH_2{-}NR^1R^2 \end{array}$$

wherein:

Ar is phenyl or thienyl; and $R^1$ and $R^2$ are independently selected from $C_1$-$C_3$ alkyl and phen($C_1$-$C_3$) alkyl, comprising:

(a) reacting an achiral Mannich ketone having the formula

$$Ar-\overset{\overset{\displaystyle O}{\|}}{C}-CH_2-CH_2-NR^1R^2$$

wherein:

Ar, $R^1$ and $R^2$ are as defined above with a complex afforded by reacting lithium aluminum hydride with (2R,3S)-(-)4-dimethylamino-1,2-diphenyl-3-methyl-2-butanol in toluene; and

b) recovering therefrom said chiral ß-amino alcohol.

**2.** The process according to Claim 1 wherein said aromatic solvent is toluene.

**3.** The process according to Claim 1 wherein said complex includes powdered molecular sieves.

**4.** The process according to Claim 1 wherein Ar is thienyl and $R^1$ and $R^2$ are $C_1$-$C_3$ alkyl.

**5.** The process according to Claim 4 wherein $R^1$ and $R^2$ are methyl.

**6.** A process for preparing S-(+)-N-methyl-3-(1-naphthalenyloxy)-3-(2-thienyl)propanamine maleate comprising:

a) reacting 2-acetylthiophene with dimethylamine hydrochloride and formaldehyde to afford 3-dimethylamino-1-(2-thienyl)-1-propanone which is recovered as an acid addition salt; and

b) reducing the compound afforded in (a) with a suspension complex of LAH:lcb to afford (S)-(+) N,N-dimethyl-3-(2-thienyl)-3-hydroxy-propanamine which is recovered as an acid addition salt; and

c) reacting the compound afforded in (b) with a 1-halonaphthalene to afford (S)-(+)-N,N-dimethyl-3-(1-naphthalenyloxy)-3-(2-thienyl)propanamine which is recovered as an acid addition salt; and

d) reacting the compound afforded in (c) with ROCOC1 where R is phenyl, benzyl, ethyl, vinyl, 1-chloroethyl or 2,2,2-trichloroethyl, in the presence of a releasing reagent to afford (S)-(-)-N-methyl-3-(1-naphthalenyloxy)-3-(2-thienyl)-propanamine which is recovered as an acid addition salt; and

e) reacting the compound afforded in (d) with maleic acid in ethyl acetate to afford (S)-(+)-N-methyl-3-(1-naphthalenyloxy)-3-(2-thienyl)-propanamine which is recovered as the maleate salt.

**7.** The process according to Claim 6 wherin R is 2,2,2-trichloroethyl.

**8.** The process according to Claim 6 wherein said releasing agent is a mixture of zinc and formic acid.